# EUROPEAN PATENT APPLICATION

(11) **EP 4 365 161 A1**
(43) Date of publication of application: **08.05.2024**
(21) Application number: 22833328.2
(22) Date of filing: 01.07.2022
(51) Int. Cl.: C07C 67/52, B01D 9/02, C07C 69/82

(54) **METHOD FOR PRODUCING CRYSTALS OF BIS-2-HYDROXYETHYL TEREPHTHALATE AND APPARATUS FOR PRODUCING CRYSTALS OF BIS-2-HYDROXYETHYL TEREPHTHALATE**

(30) Priority: 01.07.2021 JP 2021109859
(71) Applicant: Tsukishima Kikai Co., Ltd., Tokyo 104-0053 (JP)
(72) Inventor: NISHIZAWA Daisuke, Tokyo 104-0053 (JP); OGAWA Ayako, Tokyo 104-0053 (JP); FUJIWARA Taku, Tokyo 104-0053 (JP); SUZUKI Kenji, Yachiyo-city, Chiba 276-0022 (JP)
(74) Representative: Müller-Boré & Partner Patentanwälte PartG mbB
(86) International application number: PCT/JP2022/026471
(87) International publication number: WO 2023/277183

(57) **Abstract**

A method for producing crystals of bis-2-hydroxyethyl terephthalate of the present invention includes performing a step of, with use of a crystallizer (10) that performs a crystallization operation of an aqueous solution including 20% to 30% by weight of BHET and a vacuum apparatus (32) that depressurizes the inside of the crystallizer (10), obtaining a slurry (13) including crystals of BHET in the crystallizer (10) by adiabatically cooling the aqueous solution by evaporating water which is a solvent of the aqueous solution; and a step of obtaining the crystals of BHET and a mother liquid by supplying the slurry (13) discharged from the crystallizer (10) to a solid-liquid separator (20).

## Description

### [Technical Field]

The present invention relates to a method for producing crystals of bis-2-hydroxyethyl terephthalate (hereinafter referred to as "BHET") and an apparatus for producing crystals of bis-2-hydroxyethyl terephthalate.

Priority is claimed on Japanese Patent Application No. 2021-109859, filed July 1, 2021, the content of which is incorporated herein by reference.

### [Background Art]

Generally, as a method for obtaining crystals from an aqueous solution, there is, for example, a method referred to as cooling crystallization that utilizes a difference in solubility of a solute in water due to a difference in temperature between a start and an end of crystallization. In particular, in indirect cooling type crystallization, a crystallizer equipped with a cooling jacket has been industrially widely used. However, in a case where an indirect cooling crystallizer with a cooling jacket is to be applied to a large-scale facility, it is necessary to increase the diameter or the height of the crystallizer, or both the diameter and the height in order to secure a heat transfer area required for cooling. Therefore, a slurry holding capacity inside the crystallizer becomes excessive with respect to the heat transfer area. Thus, the apparatus cost, the construction cost, or the installation area also becomes excessive.

Moreover, in indirect cooling type crystallization, in the case of a configuration that includes a circulation system with a cooler outside the crystallizer that cools the process liquid, it is necessary to adjust a temperature difference between a refrigerant and a process liquid to be low to suppress scaling within the cooler. In a case where this configuration is to be applied to a large-scale facility, a cooler and a circulation pump that circulates a process liquid become excessive in order to obtain a heat exchange quantity required for cooling. Thus, the apparatus cost, the construction cost, or the installation area also becomes excessively high.

In the case of the indirect cooling type crystallization, crystal scaling or crystal deposition on a heat transfer surface over time is unavoidable, regardless of whether the configuration is using a cooling jacket or using a circulation system having a cooler. In order to remove the crystal scaling or the crystal deposition, a dissolution operation is required in a state where production is stopped. Therefore, it is difficult to avoid the production stoppage during periods other than regular maintenances.

In addition, in the cooling crystallization, both a batch operation and a continuous operation are widely used. For example, Patent Document 1 describes a crystallizing method for crystals of BHET, in which an aqueous BHET solution is cooled under a predetermined cooling rate condition, and cooled by a batch operation using a difference in solubility of BHET in water at a difference in operation temperature between the start and the end of crystallization.

Generally, as compared to the continuous operation, the batch operation in industrial processes may have disadvantages such as (1) an increase in human workload for operation due to occurrence of a start and a stop for each unit operation, (2) low flexibility in responding to an increase and a decrease in production, and (3) tendency of increasing apparatus size. In this respect, the crystallization process is not excepted. That is, the batch operation in the crystallization process has the same disadvantages as compared to the continuous operation in the crystallization process.

### [Citation List]

### [Patent Document]

[Patent Document 1]
Japanese Unexamined Patent Application, First Publication No. 2008-88096

### [Summary of Invention]

### [Technical Problem]

The present invention has been made under such a background, and thus provides a method for producing crystals of BHET, in which the method reduces an apparatus capacity, construction cost, an installation area, and the like, as compared to methods in the related art, reduces the frequency of a dissolution operation of scaling, and is capable of stably obtaining high-purity crystals of BHET from an aqueous BHET solution.

### [Solution to Problem]

In order to solve the problem, thus accomplishing such an object, the present invention proposes the following aspects.

A first aspect of the present invention is a method for producing crystals of BHET, the method including a step of, with use of a crystallizer that performs a crystallization operation of an aqueous solution including 20% to 30% by weight of BHET and a vacuum apparatus that depressurizes the inside of the crystallizer, obtaining a slurry including crystals of BHET in the crystallizer by adiabatically cooling the aqueous solution by evaporating water which is a solvent of the aqueous solution; and a step of obtaining the crystals of BHET and a mother liquid by supplying the slurry discharged from the crystallizer to a solid-liquid separator.

According to the first aspect, crystals of BHET are precipitated in a case where an aqueous BHET solution to be subjected to a crystallization operation in the crystallizer is subjected to an adiabatic cooling operation by evaporating water as a solvent using the vacuum apparatus under reduced pressure. Furthermore, it is possible to obtain high-quality purified BHET by discharging the slurry generated in the crystallizer therefrom and separating the slurry into the crystals and a mother liquid by solid-liquid separation.

A second aspect of the present invention is the method for producing crystals of BHET of the first aspect, in which the slurry is continuously obtained in the crystallizer by continuously supplying the aqueous solution in which BHET is dissolved to the crystallizer at a temperature of 60°C or higher, and adiabatically cooling the aqueous solution under conditions of 3.7 to 8.6 kPaA and 30°C to 45°C inside the crystallizer.

According to the second aspect, it is possible to crystallize favorable needle crystals of BHET. By continuously generating the crystals of BHET, it is possible to expect labor saving, improvement in productivity, improvement of product quality, reducing installation area of apparatus, and the like.

A third aspect of the present invention is the method for producing crystals of BHET according to the first or second aspect, in which the solid-liquid separator is a centrifuge or a filtration machine.

According to the third aspect, it is possible to easily obtain crystals of BHET from the slurry of BHET.

A fourth aspect of the present invention is the method for producing crystals of BHET according to any one of the first to third aspects, in which a cake layer of the crystals of BHET formed in the solid-liquid separator by solid-liquid separation is subjected to washing with water to increase a purity of BHET.

According to the fourth aspect, it is possible to easily increase the purity of the crystals of BHET.

A fifth aspect of the present invention is an apparatus for producing crystals of BHET, the apparatus including a crystallization device equipped with a crystallizer that performs a crystallization operation of an aqueous solution including 20% to 30% by weight of BHET and a vacuum apparatus that depressurizes the inside of the crystallizer, where a slurry including crystals of BHET is obtained in the crystallizer by adiabatically cooling the aqueous solution by evaporating water which is a solvent of the aqueous solution; and a solid-liquid separator where the crystals of BHET and a mother liquid are obtained from the slurry discharged from the crystallizer.

According to the fifth aspect, the crystals of BHET are precipitated in a case where the aqueous BHET solution to be subjected to a crystallization operation in the crystallizer is subjected to an adiabatic cooling operation by evaporating water as a solvent using the vacuum apparatus under reduced pressure. Furthermore, it is possible to obtain high-quality purified BHET by discharging the slurry generated in the crystallizer from the crystallizer and separating the slurry into the crystals and a mother liquid by solid-liquid separation.

### [Advantageous Effects of Invention]

According to the present invention, it is possible to reduce an apparatus capacity, construction cost, an installation area, and the like, as compared with methods in the related art; reduce the frequency of a dissolution operation of scaling; and stably obtain high-purity crystals of BHET from an aqueous BHET solution. More specifically, the aqueous BHET solution can be cooled without increasing the diameter or the height of the crystallizer, or both the diameter and the height in order to secure the heat transfer area, and a cooling energy quantity is reduced. In addition, crystal scaling or crystal deposition on the heat transfer surface over time can be suppressed, and therefore, production stoppage during periods other than regular maintenances, caused by the dissolution operation of the scaling, can also be minimized.

### [Brief Description of Drawings]

FIG. 1 is a schematic view showing a configuration of an apparatus used in an embodiment.
FIG. 2 is a graph showing a change in amount of BHET dissolved over temperatures.
FIG. 3 is a schematic view showing a configuration of an apparatus used in Example 1.
FIG. 4 is a schematic view showing a configuration of an apparatus used in Comparative Example 1.

### [Description of Embodiments]

Hereinafter, the present invention (a method for producing crystals of BHET and an apparatus for producing crystals of BHET) will be described with reference to the drawings, based on suitable embodiments.

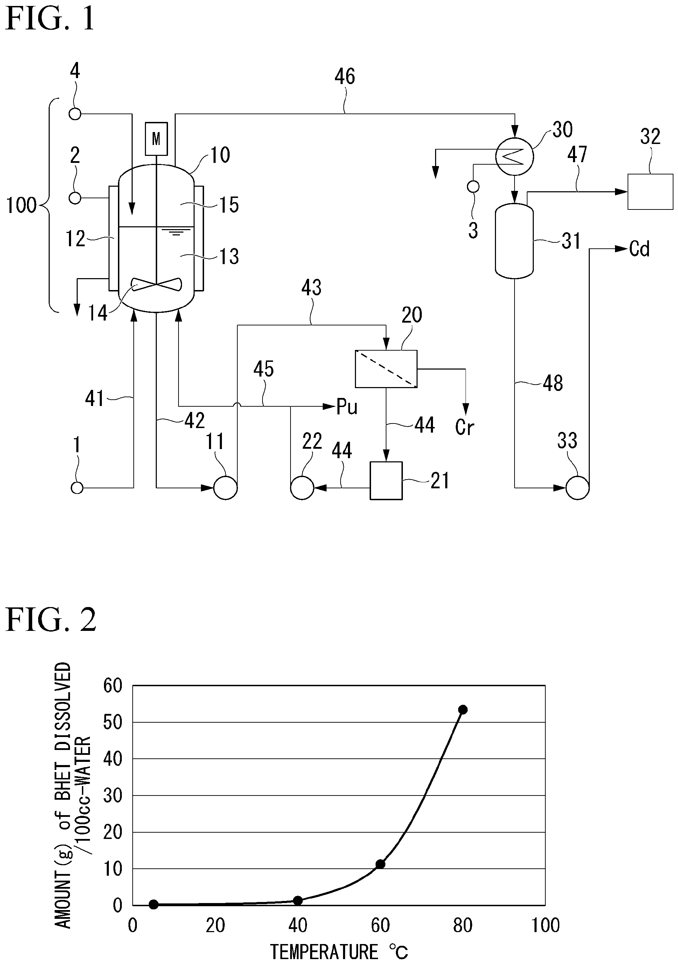

As shown in FIG. 1, the apparatus used in the embodiment generally includes a crystallizer 10, a solid-liquid separator 20, and a vacuum apparatus 32. Among these, the crystallization device 100 used for an adiabatic cooling type crystallization operation is equipped with the crystallizer 10 and the vacuum apparatus 32.

### <Crystallization Step>

An aqueous solution including BHET is supplied as a stock liquid 1 from a pipeline 41 to the crystallizer 10. A jacket 12 through which a heating medium 2 flows is provided in the periphery of the crystallizer 10. By allowing the heating medium 2 to flow through the jacket 12, the temperature of the aqueous solution in the crystallizer 10 can be maintained at a predetermined value.

In the initial stage of the crystallization step, in a case where the inside of the crystallizer 10 is depressurized using a vacuum apparatus 32 in a state where the aqueous solution including BHET is accommodated in the crystallizer 10, water included in the aqueous solution as a solvent evaporates, water vapor is further adiabatically expanded, and the temperature is lowered. By such an adiabatic cooling operation, the aqueous solution in the crystallizer 10 is cooled. By performing the adiabatic cooling type crystallization by adiabatically cooling the aqueous solution in the crystallizer 10, the heat quantity of the stock liquid 1 to be supplied and the crystallization heat of BHET are taken away with the evaporation of water. Therefore, crystals of BHET in the crystallizer 10 are precipitated, and thus, a slurry 13 in which the aqueous solution and the crystals of BHET are mixed with each other is generated. At this time, the heat quantity which is externally input as required is small. Therefore, hot water, steam, or the like may be used as the heating medium 2.

After becoming a state where the slurry 13 is kept in the crystallizer 10, the aqueous solution is newly supplied, the adiabatic crystallization is continuously performed, and a part of the slurry 13 is discharged from the crystallizer 10.

In the crystallization operation, crystals are entrained in droplets during the evaporation process of water, and thus, the crystals may be deposited as a scaling near a liquid surface of the slurry 13, such as a side wall surface of the crystallizer 10, or a drive shaft and the like of an agitator 14 in a case where the agitator 14 is installed. In the case of the indirect cooling type, it is difficult to perform a dissolution operation during the crystallization operation in a case where scaling occurs on the cooled heat transfer surface. In a case of the adiabatic cooling type crystallization, since the evaporation of water due to reduced pressure is unlikely to be hindered even in a case where the dissolution operation is carried out near the liquid surface of the slurry 13, a crystal production operation by the crystallization operation can be carried out while the scaling dissolution step is performed.

In order to dissolve the scaling, for example, a method in which a dissolution facility such as a shower ring and a spray nozzle is installed in a space 15 above the liquid surface of the slurry 13 inside the crystallizer 10, and a scaling dissolution liquid 4 such as water is supplied to a location where scaling has occurred, such as a side wall surface of the crystallizer 10, a liquid surface of the slurry 13, and a drive shaft of the agitator 14, may be considered.

Furthermore, in a case of the adiabatic cooling type crystallization, the slurry 13 can be cooled by an evaporative latent heat of water. Therefore, in a case where the apparatus of the embodiment is to be applied to a large-scale facility, it is not necessary to increase the diameter or the height of the crystallizer 10, or both the diameter and the height in order to secure a required heat transfer area. In addition, the holding capacity of the slurry 13 inside the crystallizer 10 does not become excessive.

As a result, it is possible to reduce the apparatus cost and the construction cost. In addition, it is possible to achieve compactness in a required installation area of the apparatus. Furthermore, there is no need for a dissolution operation due to production stoppage during periods other than regular maintenances, which is caused by crystal scaling over time or crystal deposition as seen in a case of the indirect cooling type. By continuously generating the crystals, labor can be saved. Constant monitoring of automatic processes is also easy. As a result, it is possible to expect improvement of productivity, improvement of product quality or safety, space saving of apparatus, and the like.

The temperature of the stock liquid 1 to be supplied to the crystallizer 10 and the temperature of the aqueous solution or the slurry 13 accommodated in the crystallizer 10 are preferably set according to the amount of BHET dissolved. The amount of BHET dissolved in 100 cc (100 cm³) of water is as follows.

5°C: 0.28 g
40°C: 1.34 g
60°C: 11.26 g
80°C: 53.42 g

A plot of the amount (g/100 cc-water) of BHET dissolved is shown in FIG. 2. In a case where the temperature of the aqueous solution is 40°C or lower or around 40°C, the amount of BHET dissolved in water is as small as about 1% by weight, and a high recovery rate of BHET is expected. In addition, in a case where the crystallization operation is performed at an internal temperature of the crystallizer 10 of 40°C or lower, it is preferable that the stock liquid 1 of the starting raw material is used as an aqueous solution including 20% to 30% by weight of BHET such that the concentration of the slurry 13 of BHET obtained in the crystallizer 10 is 20% to 30% by weight.

The aqueous solution that is supplied to the crystallizer 10 as the stock liquid 1 is preferably heated to a temperature of 65°C or higher, and preferably 65°C to 70°C, so that the entire BHET is dissolved therein. The step of supplying the stock liquid 1 to the crystallizer 10 may be of a continuous type or a batch type. The temperature of the cooled aqueous solution by adiabatic cooling in the crystallizer 10 may be controlled to around 40°C, preferably in a range of 30°C to 45°C, and more preferably in a range of 35°C to 40°C, as described above.

The crystals of BHET are precipitated in the crystallizer 10 and the slurry 13 mixed with the aqueous solution is generated. In this case, the temperature of the slurry 13 can be considered to be the same as the temperature of the aqueous solution included in the slurry 13. The temperature of the slurry 13 may temporarily rise as the stock liquid 1 is supplied to the crystallizer 10. It is preferable to discharge the slurry 13 from the crystallizer 10 in a state where the inside of the crystallizer 10 is operated at the above-described temperature by adiabatically cooling the slurry 13. In addition, the temperature of the slurry 13 is preferably controlled to be continuously maintained within the above-described temperature range.

The pressure (absolute pressure: kPaA) in the crystallizer 10 during the adiabatic cooling operation is preferably in an appropriate range with respect to the saturated water vapor pressure. For example, in a case where the temperature of the slurry 13 in the crystallizer 10 is 30°C, the pressure is preferably approximately 3.7 kPaA; in a case where the temperature is 35°C, the pressure is preferably approximately 5.0 kPaA; in a case where the temperature is 40°C, the pressure is preferably approximately 6.6 kPaA; and in a case where the temperature is 45°C, the pressure is preferably approximately 8.6 kPaA. For example, the pressure in the crystallizer 10 may be approximately 80% to 95% of the saturated water vapor pressure.

The pressure in the crystallizer 10 in the crystallization step may be controlled by controlling the pressure of a gas phase in the space 15 above the liquid surface of the slurry 13 in the inside of the crystallizer 10, and may be controlled by controlling a pressure generated by the vacuum apparatus 32 in the outside of the crystallizer 10. In a case where the pressure is measured outside the crystallizer 10, the pressure may be measured in a pipeline 46 between the crystallizer 10 and a vapor condenser 30, and may be measured in a pipeline 47 between a gas-liquid separation tank 31 and the vacuum apparatus 32.

It is preferable that the slurry 13 of BHET is continuously obtained in the crystallizer 10 by continuously supplying an aqueous solution in which BHET is dissolved at a temperature of 60°C or higher as the stock liquid 1 to the crystallizer 10, and adiabatically cooling the slurry 13 under conditions of 3.7 to 8.6 kPaA and 30°C to 45°C inside the crystallizer 10. Furthermore, it is more preferable that the adiabatic cooling crystallization is performed under the conditions of 5.0 to 6.6 kPaA and 30°C to 45°C, and preferably under the conditions of 5.0 to 6.6 kPaA and 35°C to 40°C to continuously obtain the slurry 13 of BHET.

In the crystallizer 10, the slurry 13 including an aqueous BHET solution and crystals can be obtained. The step of discharging the slurry 13 from the crystallizer 10 may be of a continuous type or of a batch type. Specifically, as described later, at least a part of the mother liquid obtained by performing solid-liquid separation on the crystals and the mother liquid from the slurry 13 may be returned to the crystallizer 10. The step of returning a mother liquid 44 to the crystallizer 10 may be of a continuous type or a batch type.

The supply of the stock liquid 1 to the crystallizer 10, the discharge of the slurry 13, and the return of the mother liquid 44 may be each continuously carried out. By performing the continuous crystallization, BHET can be continuously crystallized while maintaining the temperature of the slurry 13 and the concentration of BHET in the crystallizer 10 within predetermined ranges. The ratio between the stock liquid 1 in the crystallizer 10 and the mother liquid 44 returned to the crystallizer 10 may be appropriately adjusted. Although not particularly shown, the slurry 13 in the crystallizer 10 may be agitated by installing an agitating device such as an agitating blade inside the crystallizer 10 or by installing a circulation unit for discharging the mother liquid to the outside and supplying the mother liquid again.

### <Solid-Liquid Separation Step>

The generated slurry 13 is discharged from the crystallizer 10 via the pipeline 42 by a pump 11 and transferred to the solid-liquid separator 20 through a pipeline 43. The slurry 13 is separated into crystals of BHET and a mother liquid by supplying the slurry 13 to the solid-liquid separator 20 to perform a solid-liquid separation operation. As a result, high-quality purified BHET can be obtained.

The solid-liquid separator 20 is not particularly limited, but is preferably a centrifuge or a filtration machine. Accordingly, crystals of BHET can be easily obtained from the slurry of BHET. The centrifuge as a specific example of the solid-liquid separator 20 may be a batch-type bottom discharge centrifuge having a filter material such as a filter cloth and a metal screen, a basket-type continuous centrifuge having a metal screen, or a horizontal centrifuge. In addition, the filtration machine as a specific example of the solid-liquid separator 20 may be a vacuum filtration machine that supplies a slurry onto a continuously traveling filter cloth and performs continuous vacuum filtration by suction from a vacuum tray that reciprocates along the traveling direction of the filter cloth, a pressure filtration machine, a suction filtration machine, a centrifugal filtration machine, or the like, or may also be a sedimentation separator or the like.

After the crystals are washed in the solid-liquid separator 20, the crystals are preferably separated into a crystal portion Cr and the mother liquid 44. A method for washing the crystals is not particularly limited, but it is preferable that a cake layer formed of the crystals of BHET formed in the solid-liquid separator 20 is subjected to washing with water. As a result, the purity of the crystals of BHET can be easily increased. For efficient washing of the cake layer, it is preferable to use a solid-liquid separator having a mechanism capable of fully automatically washing the cake layer with water. The washing water used for washing the crystals may be combined with the mother liquid or may be separated from the mother liquid.

The crystals of BHET obtained by the solid-liquid separator 20 are transferred to a subsequent step as the crystal portion Cr from the solid-liquid separator 20. Higher-quality BHET can be obtained by removing adhered water from the crystal portion Cr by a drying unit. The drying unit is not particularly limited, but examples thereof include drying under reduced pressure and drying by heating.

The mother liquid 44 discharged from the solid-liquid separator 20 can be temporarily stored in a mother liquid receiving tank 21, then discharged by a mother liquid pump 22, and returned to the crystallizer 10 through a pipeline 45. As described above, the mother liquid included in the slurry 13 discharged from the crystallizer 10 is returned to the crystallizer 10, and circulated after undergoing solid-liquid separation. In a case where the accumulation or concentration of impurities in the mother liquid affects the properties or quality of crystals, a part of the mother liquid 44 may be purged and discharged to the outside of the system as a purge flow Pu.

### <Condensation Step>

Evaporation vapor generated by the adiabatic cooling operation in the crystallizer 10 passes through the pipeline 46, is condensed in the vapor condenser 30, and is guided to the gas-liquid separation tank 31. The vapor condenser 30 is arranged between the crystallizer 10 and the vacuum apparatus 32. The gas-liquid separation tank 31 is arranged between the vapor condenser 30 and the vacuum apparatus 32.

The apparatus or the steps of the vapor condenser 30 are not limited, but examples thereof include a shell-and-tube type heat exchanger, a spiral type heat exchanger, and a plate type heat exchanger. The evaporation vapor is guided to the vapor condenser 30 through which a refrigerant 3 has flowed, and achieves condensation of moisture. Due to the condensation of moisture in the evaporation vapor, a liquid phase part including condensed water and a gas phase part including a gas such as air are mixed.

In the gas-liquid separation tank 31, the gas phase part passes through the pipeline 47 and is taken in by the vacuum apparatus 32. As the vacuum apparatus 32, a model, which has a less influence on the operation even in a case where uncondensed moisture is included on the intake side, is suitable. The liquid phase part passes through the pipeline 48, is discharged by the pump 33, and is discarded or reused as a condensed water flow Cd. Since the component of the condensed water is water evaporated in the crystallizer 10, it is possible to reuse the condensed water as water for industrial use, such as washing water. The condensed water may be reused inside the system of the device or may be reused outside the system. In addition, the obtained condensed water can be supplied as a washing liquid of the solid-liquid separator 20 or the scaling dissolution liquid 4 of the crystallizer 10.

The present invention has hitherto been described, based on the suitable embodiments. However, the present invention is not limited to the above-described embodiments and various modifications can be made within a scope not departing from the concept of the present invention.

BHET used as a raw material is not particularly limited, but can be obtained by an esterification reaction between terephthalic acid and ethylene glycol, a transesterification reaction between a terephthalic acid ester such as dimethyl terephthalate, and ethylene glycol, an addition reaction between terephthalic acid and ethylene oxide, a depolymerization reaction of polyethylene terephthalate (PET) by ethylene glycol, or the like.

### [Examples]

Hereinafter, the present invention will be described with reference to specific Examples.

### (Example 1)

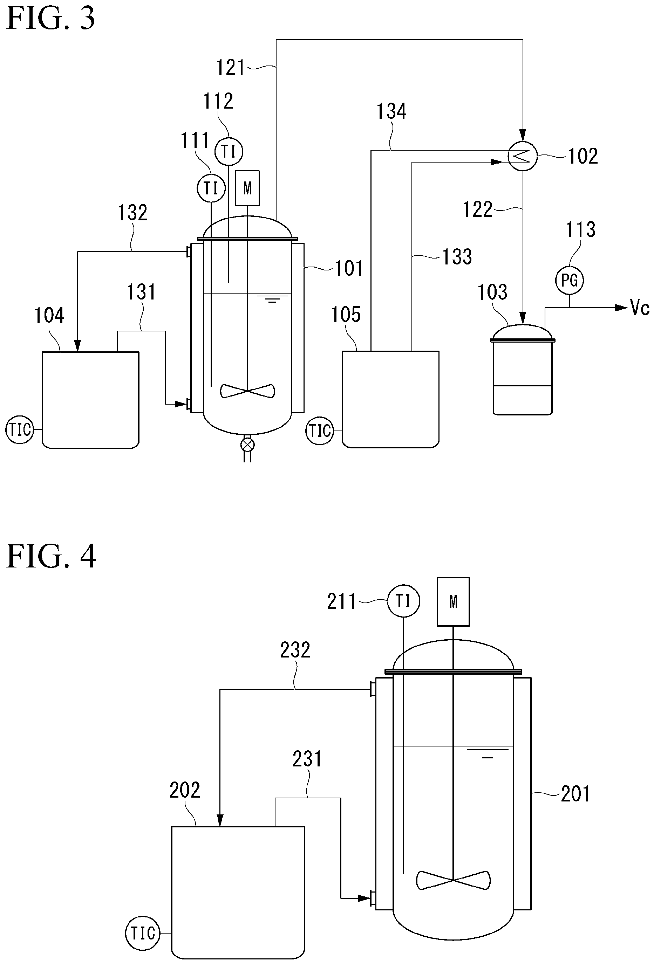

FIG. 3 shows a configuration of a device used in Example 1. A crystallizer 101 and a vapor condenser 102 are connected to each other via a pipeline 121. In addition, the vapor condenser 102 and a vacuum pump Vc are connected to each other via a pipeline 122. A gas-liquid separation tank 103 is arranged between the vapor condenser 102 and the vacuum pump Vc. The temperatures of thermostatic baths 104 and 105 are controlled by a temperature indicating controller (TIC).

First, 1.5 kg of an aqueous solution including 28% by weight of BHET (raw material) was heated to 70°C. It was visually confirmed that the solid matter in the raw material was completely dissolved in water at 70°C. Next, the entire amount of the raw material was put into the crystallizer 101. After the supply liquid of the raw material was put into the crystallizer 101, the agitator was started to work using a motor (M) and the agitating state was maintained. Hot water was allowed to pass through a jacket part of the crystallizer 101 from the thermostatic bath 104, and circulated through pipelines 131 and 132.

Next, chiller water was allowed to flow from the thermostatic bath 105 to the vapor condenser 102 and circulated through the pipelines 133 and 134. The vacuum pump Vc was started to work to adjust the pressure and the temperature. Next, while adjusting the degree of vacuum, the crystallizer 101 was adiabatically cooled to precipitate crystals of BHET. The cooling operation was completed at a point of time when a thermometer 111 indicating the temperature of the raw material indicated 38.6°C, a thermometer 112 indicating the temperature of the gas phase above the raw material indicated 36.6°C, and a pressure gauge 113 indicating the degree of vacuum of the vacuum pump Vc indicated 6.6 kPaA.

Next, the slurry was discharged from the crystallizer 101 and deliquored with a top-discharge type centrifuge (centrifugal effect: 750 G) for 5 minutes while maintaining the operation temperature at the time of completion of crystallization at 38.6°C. Next, the cake remaining on the filter cloth of the centrifuge was subjected to a crystal washing operation by spraying room-temperature water with a sprayer. In the crystal washing operation, water was used at a ratio of 10 parts by weight to 100 parts by weight of the solid content corresponding to a dried product of the cake layer. The cake layer after the crystal washing operation was deliquored with an upper discharge type centrifuge (centrifugal effect: 750 G) for 5 minutes.

### (Comparative Example 1)

FIG. 4 shows a configuration of an apparatus used in Comparative Example 1. First, about 1.9 kg of an aqueous BHET solution (raw material) having the same composition as that in Example 1 was heated to 70°C. It was visually confirmed that the solid matter in the raw material was completely dissolved in water at 70°C. Next, the entire amount of the raw material was put into the crystallizer 201. After the stock liquid was introduced, the agitator was started to work and the agitating state was maintained. Next, hot water was allowed to pass through a jacket part of the crystallizer 201 from the thermostatic bath 202, and circulated through pipelines 231 and 232.

Next, an indirect cooling operation was performed by adjusting the set temperature of a thermostatic bath 202. The temperature inside the crystallizer 201 was monitored using a thermometer 211 that indicates the temperature of the raw material. Rapid cooling was performed from 70°C to 65°C, and gradual cooling was performed from 65°C to 35°C over 3 hours. At a point of time of the temperature reaching 35°C, the operation state was maintained for 30 minutes. After completion of the cooling operation, the slurry was discharged from the crystallizer 201, and thereafter, the solid-liquid separation operation and the crystal washing operation were performed under the same conditions as in Example 1.

### (Analysis Results)

Table 1 shows the analysis results of the washed crystals of Example 1 and Comparative Example 1. The analysis items are a crystal moisture percentage by a Karl Fischer moisture meter, and a color (an L, value, an a value, and a b value) by whiteness measurement.

**[Table 1]**

| Sample | | Example 1 | Comparative Example 1 |
|---|---|---|---|
| Moisture [% by weight] | | 5.9 | 8.6 |
| Color | L | 96.2 | 94.1 |
| | a | 0.01 | 0.02 |
| | b | 0.58 | 0.63 |

According to Table 1, Example 1 (adiabatic cooling type) has a lower moisture value than that in Comparative Example 1 (indirect cooling type), and it can be expected to reduce a load of the drying process in the subsequent stage. In addition, the washed crystals of BHET obtained in Example 1 exhibited a chromaticity that was equal to or higher than that in Comparative Example 1.

Tables 2 and 3 show material balances based on the test results of each of Example 1 and Comparative Example 1. The total amount of the supply liquid is converted to 100.

**[Table 2]**

| Example 1 | Supply liquid | Vapor | Slurry | Crystals | Mother liquid |
|---|---|---|---|---|---|
| Weight | | | | | |
| BHET (1) | 26.6 | 0 | 1.0 | 0 | 1.0 |
| Impurity | 1.4 | 0 | 1.4 | 0.1 | 1.3 |
| Water | 72.0 | 7.7 | 64.3 | 2.5 | 61.8 |
| Liq. Total | 100.0 | 7.7 | 66.7 | 2.6 | 64.1 |
| BHET (s) | 0 | 0 | 25.6 | 23.5 | 2.0 |
| Slurry Total | 100.0 | 7.7 | 92.3 | 26.2 | 66.1 |

**[Table 3]**

| Comparative Example 1 | Supply liquid | Slurry | Crystals | Mother liquid |
|---|---|---|---|---|
| Weight | | | | |
| BHET (1) | 26.6 | 1.1 | 0 | 1.1 |
| Impurity | 1.4 | 1.4 | 0 | 1.4 |
| Water | 72.0 | 72.0 | 2.5 | 69.5 |
| Liq. Total | 100.0 | 74.5 | 2.6 | 71.9 |
| BHET (s) | 0 | 25.5 | 23.4 | 2.0 |
| Slurry Total | 100.0 | 100.0 | 26.0 | 74.0 |

In a case of comparing the material balances between Example 1 and Comparative Example 1, it can be seen that in Example 1 (adiabatic cooling type), a part of the moisture in the supply liquid is evaporated as a vapor, and the amount of the mother liquid in the slurry decreases accordingly. Since the component of the condensate is moisture evaporated in the crystallizer, the condensate can be reused as water for industrial use such as washing water, which leads to a reduction in a basic unit of a plant. In addition, since the amount of the mother liquid is small, it leads to a reduction in the load of a regeneration process or the load of a waste water treatment facility in a case of recycling the mother liquid.

In a case where the crystallizers were sized based on the results of Example 1 and Comparative Example 1, the slurry capacity in the crystallizer in Table 2 (Example 1) can be suppressed to be 114 or less of that of the crystallizer in Table 3 (Comparative Example 1). Examples of advantages of the reduction in capacity of the crystallizer include a reduction in miscellaneous time such as time taken for inflow of a liquid at the start of operation and discharge of the liquid at the stop of the operation, a reduction in capacity of a motor installed in the crystallizer, and a reduction in apparatus cost or construction cost associated with downsizing of the crystallizer.

### [Industrial Applicability]

The present invention can be applied to a method for producing crystals of BHET or an apparatus for producing crystals of BHET, using a crystallizer that performs a crystallization operation of an aqueous solution including BHET and a vacuum apparatus that depressurizes the inside of the crystallizer.

### [Reference Signs List]

100: Crystallization device
10, 101, 201: Crystallizer
13: Slurry
20: Solid-liquid separator
30, 102: Vapor condenser
31, 103: Gas-liquid separation tank
32: Vacuum apparatus

## Claims

1. A method for producing crystals of bis-2-hydroxyethyl terephthalate, the method comprising:
a step of, with use of a crystallizer that performs a crystallization operation of an aqueous solution including 20% to 30% by weight of bis-2-hydroxyethyl terephthalate and a vacuum apparatus that depressurizes an inside of the crystallizer, obtaining a slurry including crystals of bis-2-hydroxyethyl terephthalate in the crystallizer by adiabatically cooling the aqueous solution by evaporating water which is a solvent of the aqueous solution; and
a step of obtaining the crystals of bis-2-hydroxyethyl terephthalate and a mother liquid by supplying the slurry discharged from the crystallizer to a solid-liquid separator.

2. The method for producing crystals of bis-2-hydroxyethyl terephthalate according to Claim 1,
wherein the slurry is continuously obtained in the crystallizer by continuously supplying the aqueous solution in which bis-2-hydroxyethyl terephthalate is dissolved at a temperature of 60°C or higher to the crystallizer, and adiabatically cooling the aqueous solution under conditions of 3.7 to 8.6 kPaA and 30°C to 45°C inside the crystallizer.

3. The method for producing crystals of bis-2-hydroxyethyl terephthalate according to Claim 1 or 2,
wherein the solid-liquid separator is a centrifuge or a filtration machine.

4. The method for producing crystals of bis-2-hydroxyethyl terephthalate according to any one of Claims 1 to 3,
wherein a cake layer of the crystals of bis-2-hydroxyethyl terephthalate formed in the solid-liquid separator by solid-liquid separation is subjected to washing with water to increase a purity of bis-2-hydroxyethyl terephthalate.

5. An apparatus for producing crystals of bis-2-hydroxyethyl terephthalate, the apparatus comprising:
a crystallization device equipped with a crystallizer that performs a crystallization operation of an aqueous solution including 20% to 30% by weight of bis-2-hydroxyethyl terephthalate and a vacuum apparatus that depressurizes an inside of the crystallizer, where a slurry including crystals of bis-2-hydroxyethyl terephthalate is obtained in the crystallizer by adiabatically cooling the aqueous solution by evaporating water which is a solvent of the aqueous solution; and
a solid-liquid separator where the crystals of bis-2-hydroxyethyl terephthalate and a mother liquid are obtained from the slurry discharged from the crystallizer.
